# EUROPEAN PATENT SPECIFICATION

(11) **EP 2 595 698 B1**
(45) Date of publication and mention of the grant of the patent: **06.09.2017**
(21) Application number: 10741925.1
(22) Date of filing: 19.07.2010
(51) Int. Cl.: A61N 1/36, A61N 5/067, A61N 5/06, A61N 1/05

(54) **COCHLEAR IMPLANT HEARING INSTRUMENT**
COCHLEAIMPLANTAT-HÖRINSTRUMENT
APPAREIL AUDITIF DE TYPE IMPLANT COCHLÉAIRE

(43) Date of publication of application: 29.05.2013
(73) Proprietor: Advanced Bionics AG, 8712 Stäfa (CH)
(72) Inventor: AMBETT, Ranjeeta, 1110 Morges (CH); YANISSE, Daniel, 1003 Lausanne (CH)
(74) Representative: Schwan Schorer & Partner mbB
(86) International application number: PCT/EP2010/060423
(87) International publication number: WO 2012/010196

(56) References cited:
- WO-A1-2010/040142
- US-A1- 2010 174 330
- K. SATORI ET AL.: "Polyimide-coated small-diameter optical fiber sensors for embedding in composite laminate structures", SMART STRUCTURES AND MATERIALS 2001: SENSORY PHENOMENA AND MEASUREMENT INSTRUMENTATION FOR SMART STRUCTURES AND MATERIALS, PROC. SPIE, vol. 4328, 20 January 2001 (2001-01-20), pages 285-294, XP002630694, DOI: 10.1117/12.435531

## Description

The present invention relates to a hearing instrument for direct stimulation of the cochlea comprising an audio signal source for providing audio signals (typically a microphone arrangement for capturing ambient sound) a sound processor for generating auditory nerve stimulation signals from the audio signals, a stimulation assembly to be implanted into the cochlea and comprising an electrode array, and a driver unit for providing the stimulation assembly, according to the auditory nerve stimulation signals from the sound processor, with electric stimulation signals for the electrode array.

Cochlear implant hearing instruments for electrical stimulation of the auditory nerve via an electrode array implanted in the cochlea are well-known since many years. Typically, the microphone arrangement and the sound processor are provided as part of an external unit to be worn at the patient's head close to the ear; the external unit may be fixed at the patient's head by magnets cooperating with implanted magnets. The auditory nerve stimulation signals are transmitted via a wireless inductive subcutaneous link to the implanted part of the hearing instrument; typically, the implanted part is powered by the external unit via a wireless inductive subcutaneous power link.

Typically, sensorineural hearing loss begins as a high-frequency loss which generally involves the basal turn of the cochlea. Such high-frequency hearing loss may be treated by electrical stimulation of the basal part of the cochlea via an implanted short electrode array. However, as the hearing loss progresses, also the low-frequencies become involved, thereby necessitating the stimulation of the apical turns of the cochlea. Treatment of the low-frequency hearing loss requires to replace the short electrode array by a longer electrode array. However, such re-intervention requires a second surgery action and may cause damage of the remnant neurons in the apical turns.

It is also known that the cochlea may be stimulated by light, see for example, AD Izzo et al., "Laser stimulation of the auditory nerve", Lasers in surgery and medicine 38, 2006; AD Izzo et al., "Selectivity of neural stimulation in the auditory system: a comparison of optic and electric stimuli", Journ. Biomed. Optics 12, 2007 ; and AD Izzo et al., "Laser stimulation of auditory neurons : effect of shorter pulse duration and penetration depth", Biophysical Journal 94, 2008. Optical stimulation results in discrete stimulation of the hair cells without diffusion or overlap.

WO 2009/072123 A2 relates to optical stimulation of the cochlea via a plurality of optical fibers implanted in the cochlea. A similar system is described in US 2006/0161227 A1.

US 6,921,413 B2 relates, in a general manner, to the direct stimulation of neural tissue with optical energy.

WO 2009/079704 A1 mentions that the cochlea may be stimulated either electrically via an implanted electrode array or optically via an optical fiber.

US 2010/0094380 A1 relates to a cochlear implant hearing instrument comprising a lead assembly implanted in the cochlea which comprises in addition to electrode contacts at least one optical contact for providing both electrical and optical stimulation to the cochlea.

US 2010/0114190 A1 mentions that the auditory nerve may be stimulated both by an electrical signal and an optical signal, in particular by using an optical fiber having a metallization layer applied to it, with the optical stimulation signal being applied through the optical fiber and the electrical stimulation signal being applied to the metallization layer.

US 2006/0129210 A1 relates to a cochlear implant comprising a primary wave guide having various output positions along the light guiding axis for optically stimulating auditory neuron sites of the cochlea. The light coupled into the primary wave guide also may be used for being transformed into an electrical stimulation signal to the cochlea.

US 2010/174330 A1 relates to a cochlear implant device, wherein the stimulation assembly implanted into the cochlea comprises electrical contacts for stimulating the basal region of the cochlea and electrical contacts for optically stimulating the medial region and/or the apical region of the cochlear, thereby avoiding the need for additional surgery in case of progressing hearing loss of the patient. The optical contacts are realized as the ends of optical fibers, with the optical fibers being completely embedded within a carrier member.

It is an object of the invention to provide for a cochlea implant hearing instrument allowing for treatment of progressive hearing loss involving also the lower frequencies, while avoiding damages of the apical part of the cochlea as far as possible.

According to the invention, this object is achieved by a hearing instrument as defined in claim 1.

The invention is beneficial in that, by stimulating the basal part of the cochlea via a short electrode array implanted within the basal part of the cochlea and stimulating the apical part of the cochlea via a plurality of optical fibers extending beyond the electrode array into the apical part of the cochlea, the high-frequency hearing loss can be treated by a conventional short electrode array in the basal part of the cochlea, while damages to the apical part of the cochlea, which may result from conventional treatment of low-frequency hearing loss by a long electrode array can be avoided due to the use of optical fibers in the apical part of the cochlea. The reason is that such arrangement of fibers for optical stimulation of the apical part of the cochlea can be realized with a significantly lower outer diameter than the tip of a classical electrical cochlea implant electrode array. For example, optical fibers having an outer diameter of about 50 µm may be used, while the diameter of the tip of a classical cochlea implant typically is about 500 µm (for example, an assembly including three of such optical fibers would have an external diameter of about 120 µm when grouped together).

Preferred embodiments of the invention are defined in the dependent claims.

Hereinafter, examples of the invention will be illustrated by reference to the attached drawings, wherein:
- Fig. 1: is a cross-sectional view of an example of a hearing instrument according to the invention after implantation;
- Fig. 2: is a block diagram of the system of Fig. 1; and
- Fig. 3: is a cross-sectional view of an example of a stimulation assembly according to the invention prior to implantation in the cochlea.

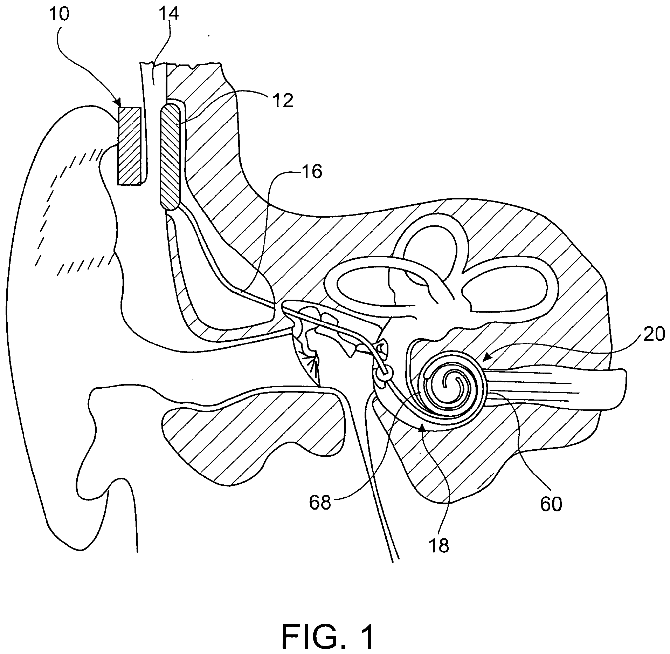

Fig. 1 shows a cross-sectional view of the mastoid region, the middle ear and the inner ear of a patient after implantation of an example of a hearing instrument according to the invention, wherein the hearing instrument is shown only schematically. The system comprises an external unit 10 which is worn outside the patient's body at the patient's head, typically at a location close to the user's ear, and an implantable unit 12 which is implanted under the patient's skin 14, usually in an artificial cavity created in the user's mastoid. The implantable unit 12 is connected, via a cable assembly 16, to a stimulation assembly 18 implanted within the cochlea 20. The external unit 10 is fixed at the patient's skin in a position opposite to the implantable unit 12, for example, by magnetic forces created between at least one fixation magnet provided in the external unit 10 and at least one cooperating fixation magnet provided in the implantable unit 12 (such magnets are not shown in Fig. 1).

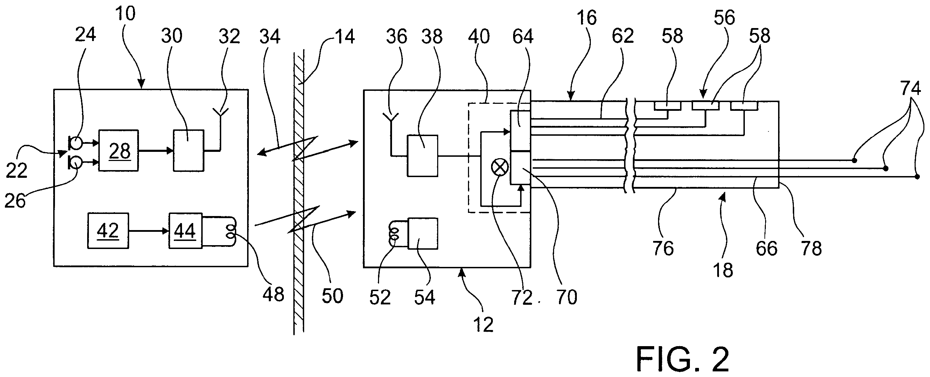

An example of a block diagram of the system of Fig. 1 is shown in Fig. 2. The external unit 10 includes a microphone arrangement 22 which typically comprises at least two spaced-apart microphones 24 and 26 for capturing audio signals from ambient sound, which audio signals are supplied to a sound processor 28 which generates auditory nerve stimulation signals as coded signals from the audio signals. The coded signals are supplied to a transmission unit 30 connected to a transmission antenna 32 in order to enable transcutaneous transmission of the stimulation signals via an inductive link 34 to the implantable unit 12 which comprises a receiver antenna 36 connected to a receiver unit 38 for receiving the transmitted stimulation signals. The received stimulation signals are supplied to a driver unit 40 which drives the stimulation assembly 18.

The external unit 10 also comprises a power supply 42, which may be a replaceable or rechargeable battery, a power transmission unit 44 and a power transmission antenna 46 for transmitting power to the implantable unit 12 via a wireless power link 50. The implantable unit 12 comprises a power receiving antenna 52 and a power receiving unit 54 for powering the implanted electronic components with power received via the power link 50.

Preferably the stimulation signal antennas 32, 36 are separated from the power antennas 48, 52 in order to optimize both the data link 34 and the power link 50. However, if a particularly simple design is desired, the antennas 32 and 48 and the antennas 36 and 52 could be physically formed by a single antenna, respectively.

The stimulation assembly 18 comprises a short electrode array 56 comprising a plurality of longitudinally aligned electrodes 58 for electrical stimulation of the auditory nerve in the basal part 60 of the cochlea 20 according to electrical stimulation signals received via wires 62 from an electrical driver 64 of the driver unit 40 (typically, a separate wire 62 is provided for each of the electrodes 58).

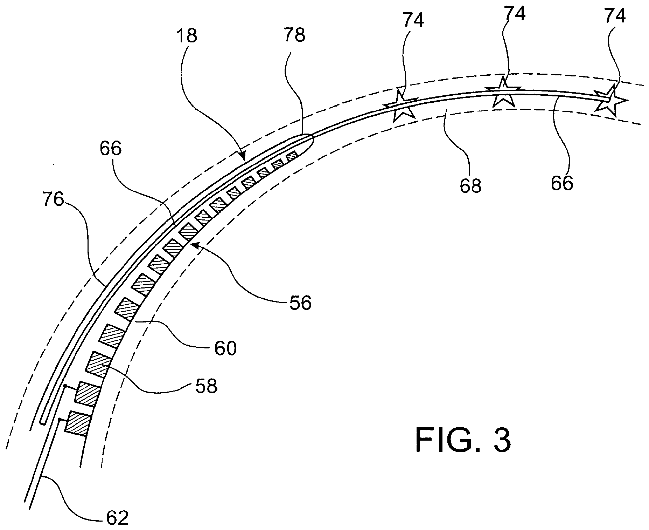

In addition to the electrode array 56, the stimulation assembly 18 also comprises a plurality of optical fibers 66 which extend beyond the electrode array 56 into the apical part 68 of the cochlea 20 for optical stimulation of the auditory nerve in the apical part 68 of the cochlea 20 with stimulation light received from an optical driver 70 of the driver unit 40 including a light source 72 (see also Fig. 3). The light source 72 may be a LED or a laser diode. Instead of a single light source a plurality of light sources may be provided. The optical driver 70 may comprise optical switches for coupling the light from the light source 72 as stimulation light into the optical fibers 66 according to the stimulation signals received from the external unit 10. Such optical switches may be realized, for example, by a spatial light modulator comprising a liquid crystal display linear array or a linear array of micro-mirrors, as described, for example in US 2006/0129210 A1. Preferably, stimulation light having the same wavelength is provided in all of the optical fibers 66.

The optical fibers are designed in such a manner that the light emission points 74, from where the stimulation light is emitted into the cochlea 20, are spaced-apart from each other along the apical part 68 of the cochlea 20. In the embodiment shown in Figs. 2 and 3 each light emission point 74 is associated to a separate one of the optical fibers 66, with each light emission point 74 being formed by the tip of the respective fiber 66, wherein the optical fibers 66 have different lengths in order to have each light emission point 74 at the desired position within the cochlea 20. Preferably, the stimulation assembly 18 comprises at least three of the optical fibers 66.

In order to prevent the apical part 68 of the cochlea 20 from damages when implanting the optical fibers 66, it is necessary to use fibers 66 having a diameter as small as possible. Preferably, the outer diameter of each fiber 66 should be less than 60 µm, with a diameter of the fiber core of less than 10 µm (for example, fibers having an external diameter of 52 µm, with a 8 µm diameter core and a 40 µm polyimide cladding are used in civil engineering, see K. Satori et al.," Development of small-diameter optical fiber sensors for damage detection in composite laminates", Proc. SPIE, Vol. 3986, 104, 2000; K. Satori et al., "Polyimide-coated small-diameter optical fiber sensors for embedding in composite laminate structures", Proc. SPIE, Vol. 4328, 285, 2001). The cladding of the fibers 66 has to be biocompatible. It is known that polyimide coating is biocompatible (see e.g. C. Yang et al., "Biocompatibility of a physiological pressure sensor", Biosensors and bioelectronics 19, 2003, and MJ Kim et al., "Biocompatible low-index cladding material for medical applications", Proc. SPIE, Vol. 5691, 154, 2005); for example, such coatings are used on commercial physiological pressure sensors.

Preferably, the stimulation light is in the wavelength range of from 1 to 2 µm, with an optical power of 0.01 to 1 J/cm². Preferably, the stimulation light is pulsed light having a pulse duration of 1 to 100 µs, with a repetition rate of 100 to 2,000 Hz. Detailed examples concerning the type of fibers and the type of light used for optical stimulation of the cochlea can be found in US 2006/0161227 A1; AD Izzo et al., "Laser stimulation of the auditory nerve", Lasers in surgery and medicine 38, 2006; AD Izzo et al., "Selectivity of neural stimulation in the auditory system: a comparison of optic and electric stimuli", Journ. Biomed. Optics 12, 2007 ; and AD Izzo et al., "Laser stimulation of auditory neurons : effect of shorter pulse duration and penetration depth", Biophysical Journal 94, 2008.

As can be seen in Figs. 2 and 3, the stimulation assembly 18 comprises a body 76 for housing the electrode array 56 and a proximal portion of the optical fibers 66, with an end portion of each fiber 66 leaving the body 76 at the distal end 78 of the body 76 (although Fig. 3 shows the stimulation assembly 18 in a condition prior to implantation into the cochlea 20, namely in a only slightly curved shape, the part of the cochlea 20 into which the stimulation assembly 18 is to be implanted is indicated schematically in Fig. 3 in dashed lines in an "unrolled" condition).

## Claims

1. A hearing instrument for direct stimulation of the cochlea (20) of a patient, comprising
an audio signal source (22) for providing audio signals,
a sound processor (28) for generating auditory nerve stimulation signals from the audio signals,
a stimulation assembly (18) to be implanted into the cochlea and comprising an electrode array (56) to be located in the basal part (60) of the cochlea for electrical stimulation of the auditory nerve in the basal part of the cochlea and a plurality of optical fibers (66) extending beyond the electrode array into the apical part (68) of the cochlea for optical stimulation of the auditory nerve in the apical part of the cochlea, and
a driver unit (40) for providing the stimulation assembly, according to the auditory nerve stimulation signals from the sound processor, with electric stimulation signals for the electrode array and with stimulation light from at least one light source (72) for the optical fibers,
**characterized in that**
the stimulation assembly (18) comprises a body (76) for housing the electrode array (56) and a proximal portion of the optical fibers (66), with an end portion of each fiber leaving the body at the distal end (78) of the body.

2. The hearing instrument of claim 1, wherein the optical fibers (66) comprise light emission points (74) for emitting the stimulation light into the apical part (68) of the cochlea (20) which are spaced apart from each other along a length of the stimulation assembly (18).

3. The hearing instrument of one of claims 1 and 2, wherein each light emission point (74) is associated to a separate one of the optical fibers (66).

4. The hearing instrument of claim 3, wherein each light emission point (74) is formed by the tip of the respective fiber (66), with the optical fibers having different lengths.

5. The hearing instrument of one of the preceding claims, wherein the stimulation assembly (18) comprises at least three of the optical fibers (66).

6. The hearing instrument of one of the preceding claims, wherein each fiber (66) comprises a cladding made of polyimide.

7. The hearing instrument of one of the preceding claims, wherein the light source (72) is for providing stimulation light having a wavelength from 1 to 2 µm and a power from 0.01 to 1 J/cm².

8. The hearing instrument of one of the preceding claims, wherein the light source (72) is for providing pulsed stimulation light having a pulse duration of 1 to 100µs and a repetition rate of 100 to 2000 Hz.

9. The hearing instrument of one of the preceding claims, wherein the outer diameter of each of the fibers (66) is less than 60 µm, and wherein the diameter of the core of each of the optical fibers (66) is less than 10 µm.

10. The hearing instrument of one of the preceding claims, wherein the light source (72) is a LED or a laser diode.

11. The hearing instrument of one of the preceding claims, wherein the audio signal source is a microphone arrangement (22) for capturing audio signals from ambient sound, wherein the microphone arrangement (22) and the sound processor (28) form part of an external unit (10) to be worn in, at or close to a patient's ear, wherein the driver unit (40) forms part of an implantable unit (12), with the external unit and the implantable unit comprising means (30, 32, 36, 38) for transmitting the auditory nerve stimulation signals via a wireless transcutaneous link (34) to the driver unit, and wherein the external unit (10) and the implantable unit (12) comprise means (44, 48, 52, 54) for transmitting power via a wireless transcutaneous inductive power link to the implantable unit.

12. The hearing instrument of one of the preceding claims, wherein the driver unit (40) comprises optical switches (70) for coupling the light from the light source (72) according to the auditory nerve stimulation signals as stimulation light into the optical fibers (66).

13. The hearing instrument of claim 12, wherein the driver unit (40) is designed to provide for stimulation light having the same wavelengths in all of the optical fibers (66).

## Patentansprüche

1. Hörgerät zur direkten Stimulation der Cochlea (20) eines Patienten, mit:
einer Audiosignalquelle (22) zum Bereitstellen von Audiosignalen,
einem Schallprozessor (28) zum Erzeugen von Hörnervstimulationssignalen aus den Audiosignalen,
einer Stimulationsbaugruppe zum Implantieren in die Cochlea, mit einer Elektrodenanordnung (56) zum Anordnen in dem basalen Teil (60) der Cochlea zwecks elektrischer Stimulation des Hörnervs im basalen Teil der Cochlea und einer Mehrzahl von optischen Fasern (66), die sich über die Elektrodenanordnung hinaus in den apikalen Teil (68) zwecks optischer Stimulation des Hörnervs im apikalen Teil der Cochlea erstrecken, und
einer Treibereinheit (40), um die Stimulationsbaugruppe gemäß den Hörnervstimulationssignalen von dem Schallprozessor mit elektrischen Stimulationssignalen für die Elektrodenanordnung und mit Stimulationslicht aus mindestens einer Lichtquelle (72) für die optischen Fasern zu versorgen,
**dadurch gekennzeichnet, dass**
die Stimulationsbaugruppe (18) einen Körper (76) aufweist, um die Elektrodenanordnung (56) und einen proximalen Abschnitt der optischen Fasern aufzunehmen, wobei ein Endabschnitt einer jeden Faser den Körper an dem distalen Ende (78) des Körpers verlässt.

2. Hörgerät gemäß Anspruch 1, wobei die optischen Fasern (66) Lichtemissionspunkte (74) zum Emittieren des Stimulationslichts in den apikalen Teil (68) der Cochlea aufweisen, welche im Abstand voneinander entlang einer Länge der Stimulationsbaugruppe (18) angeordnet sind.

3. Hörgerät gemäß einem der Ansprüche 1 und 2, wobei jeder der Lichtemissionspunkte (74) mit einer anderen der optischen Fasern (66) assoziiert ist.

4. Hörgerät gemäß Anspruch 3, wobei jeder Lichtemissionspunkt (74) von der Spitze der jeweiligen Faser (66) gebildet wird, und wobei die optischen Fasern unterschiedliche Längen aufweisen.

5. Hörgerät gemäß einem der vorhergehenden Ansprüche, wobei die Stimulationsbaugruppe (18) mindestens drei der optischen Fasern (66) aufweist.

6. Hörgerät gemäß einem der vorhergehenden Ansprüche, wobei jede Faser (66) einen Mantel aus Polyimid aufweist.

7. Hörgerät gemäß einem der vorhergehenden Ansprüche, wobei die Lichtquelle (72) zum Liefern von Stimulationslicht mit einer Wellenlänge zwischen 1 und 2 µm und einer Energie von 0,01 bis 1 J/cm² ausgebildet ist.

8. Hörgerät gemäß einem der vorhergehenden Ansprüche, wobei die Lichtquelle zum Liefern von gepulstem Stimulationslicht mit einer Pulsdauer von 1 bis 100 µs und einer Wiederholungsrate von 100 bis 2000 Hz ausgebildet ist.

9. Hörgerät gemäß einem der vorhergehenden Ansprüche, wobei der Außendurchmesser einer jeden Faser (66) weniger als 60 µm beträgt und wobei der Durchmesser des Kerns einer jeden der optischen Fasern (66) weniger als 10 µm beträgt.

10. Hörgerät gemäß einem der vorhergehenden Ansprüche, wobei es sich bei der Lichtquelle (72) um eine LED oder eine Laserdiode handelt.

11. Hörgerät gemäß einem der vorhergehenden Ansprüche, wobei es sich bei der Audiosignalquelle um eine Mikrofonanordnung (22) zum Auffangen von Audiosignalen aus Umgebungsschall handelt, wobei die Mikrofonanordnung (22) und der Schallprozessor (28) Teil einer externen Einheit bilden, die in einen, an einem oder nahe eines Ohrs des Patienten zu tragen ist, wobei die Treibereinheit einen (40) Teil einer implantierbaren Einheit (12) bildet, wobei die externe Einheit und die implantierbare Einheit Mittel (30, 32, 36, 38) zum Senden der Hörnervstimulationssignalen über eine drahtlose transkutane Verbindung (34) zu der Treibereinheit aufweisen, und wobei die externe Einheit (10) und die implantierbare Einheit (12) Mittel (44, 48, 52, 54) zum Senden von Energie über eine drahtlose transkutane induktive Energieverbindung zu der implantierbaren Einheit aufweisen.

12. Hörgerät gemäß einem der vorhergehenden Ansprüche, wobei die Treibereinheit (40) optische Schalter (70) aufweist, um das Licht aus der Lichtquelle (72) gemäß den Hörnervstimulationssignalen als Stimulationslicht in die optischen Fasern (66) einzukoppeln.

13. Hörgerät gemäß Anspruch 12, wobei die Treibereinheit (40) ausgebildet ist, um Stimulationslicht mit denselben Wellenlängen in allen optischen Fasern (66) zu liefern.

## Revendications

1. Appareil auditif pour la stimulation directe de la cochlée (20) d'un patient, comprenant :
une source de signal audio (22) pour délivrer des signaux audio,
un processeur de son (28) pour générer des signaux de stimulation du nerf auditif à partir des signaux audio,
un ensemble de stimulation (18) à implanter dans la cochlée et comprenant un réseau d'électrodes (56) à situer dans la partie basale (60) de la cochlée pour une stimulation électrique du nerf auditif dans la partie basale de la cochlée et une pluralité de fibres optiques (66) s'étendant au-delà du réseau d'électrodes dans la partie apicale (68) de la cochlée pour une stimulation optique du nerf auditif dans la partie apicale de la cochlée, et
une unité de commande (40) pour délivrer à l'ensemble de stimulation, conformément aux signaux de stimulation du nerf auditif provenant du processeur de son, des signaux de stimulation électrique pour le réseau d'électrodes et avec une lumière de stimulation provenant d'au moins une source de lumière (72) pour les fibres optiques,
**caractérisé en ce que**
l'ensemble de stimulation (18) comprend un corps (76) pour loger le réseau d'électrodes (56) et une partie proximale des fibres optiques (66), avec une partie d'extrémité de chaque fibre sortant du corps à l'extrémité distale (78) du corps.

2. Appareil auditif selon la revendication 1, dans lequel les fibres optiques (66) comprennent des points d'émission de lumière (74) pour émettre la lumière de stimulation dans la partie apicale (68) de la cochlée (20) qui sont espacés les des autres le long d'une longueur de l'ensemble de stimulation (18).

3. Appareil auditif selon l'une des revendications 1 et 2, dans lequel chaque point d'émission de lumière (74) est associé à une fibre optique distincte des fibres optiques (66).

4. Appareil auditif selon la revendication 3, dans lequel chaque point d'émission de lumière (74) est formé par la pointe de la fibre respective (66), les fibres optiques ayant des longueurs différentes.

5. Appareil auditif selon l'une des revendications précédentes, dans lequel l'ensemble de stimulation (18) comprend au moins trois des fibres optiques (66).

6. Appareil auditif selon l'une des revendications précédentes, dans lequel chaque fibre (66) comprend une gaine en polyimide.

7. Appareil auditif selon l'une quelconque des revendications précédentes, dans lequel la source de lumière (72) est destinée à délivrer une lumière de stimulation ayant une longueur d'onde de 1 à 2 µm et une puissance de 0,01 à 1 J/cm².

8. Appareil auditif selon l'une des revendications précédentes, dans lequel la source de lumière (72) est destinée à délivrer une lumière de stimulation pulsée ayant une durée d'impulsion de 1 à 100 µs et une fréquence de répétition de 100 à 2000 Hz.

9. Appareil auditif selon l'une des revendications précédentes, dans lequel le diamètre extérieur de chacune des fibres (66) est inférieur à 60 µm, et dans lequel le diamètre du coeur de chacune des fibres optiques (66) est inférieur à 10 µm.

10. Appareil auditif selon l'une des revendications précédentes, dans lequel la source de lumière (72) est une LED ou une diode laser.

11. Appareil auditif selon l'une des revendications précédentes, dans lequel la source de signal audio est un arrangement de microphone (22) pour capturer des signaux audio à partir d'un son ambiant, où l'arrangement de microphone (22) et le processeur de son (28) font partie d'une unité externe (10) destinée à être portée à l'intérieur, ou à proximité, de l'oreille d'un patient, où l'unité de commande (40) fait partie d'une unité implantable (12), avec l'unité externe et l'unité implantable comprenant des moyens (30, 32, 36, 38) pour transmettre les signaux de stimulation du nerf auditif par l'intermédiaire d'une liaison transcutanée sans fil (34) à l'unité de commande, et où l'unité externe (10) et l'unité implantable (12) comprennent des moyens (44, 48, 52, 54) pour transmettre de la puissance par l'intermédiaire d'une liaison de puissance par induction transcutanée sans fil à l'unité implantable.

12. Appareil auditif selon l'une des revendications précédentes, dans lequel l'unité de commande (40) comprend des commutateurs optiques (70) pour coupler la lumière provenant de la source de lumière (72) conformément aux signaux de stimulation du nerf auditif comme lumière de stimulation dans les fibres optiques (66) .

13. Appareil auditif selon la revendication 12, dans lequel l'unité de commande (40) est conçue pour délivrer une lumière de stimulation ayant les mêmes longueurs d'ondes dans toutes les fibres optiques (66).
